# EUROPÄISCHE PATENTSCHRIFT

(11) **EP 1 162 938 B1**
(45) Veröffentlichungstag und Bekanntmachung des Hinweises auf die Patenterteilung: **05.11.2003**
(21) Anmeldenummer: 01900386.2
(22) Anmeldetag: 04.01.2001
(51) Int. Cl.: A61K 7/06

(54) **ZUSAMMENSETZUNG FÜR EIN HAARBEHANDLUNGSMITTEL IN FORM EINES AEROSOL-SCHAUMES**
COMPOSITION FOR A HAIR TREATMENT PREPARATION IN THE FORM OF AN AEROSOL FOAM
COMPOSITION DE TRAITEMENT CAPILLAIRE SOUS FORME D'UNE MOUSSE AEROSOL

(30) Priorität: 21.01.2000 DE 10002513
(43) Veröffentlichungstag der Anmeldung: 19.12.2001
(73) Patentinhaber: Wella Aktiengesellschaft, 64274 Darmstadt (DE)
(72) Erfinder: SCHMENGER, Jürgen, 64331 Weiterstadt (DE); ABELS, Wilhelm, Simi Valley, CA 93065 (US); JAHEDSHOAR, Mehrdad, Calabasas, CA 91302 (US)
(74) Vertreter: Schulze, Rainer (DE)
(86) Internationale Anmeldenummer: EP0100032
(87) Internationale Veröffentlichungsnummer: WO01052800

(56) Entgegenhaltungen:
- EP-A- 0 680 743
- EP-A- 0 745 373
- WO-A-96/40815

## Beschreibung

Gegenstand der Erfindung ist eine Zusammensetzung für ein Haarbehandlungsmittel, welches vorzugsweise in Form eines optisch klaren, durchsichtigen oder durchscheinenden Produktes vorliegt und als Aerosol-Schaum angewendet wird. Das Mittel kann insbesondere ein haarpflegendes Mittel sein und istals Leave-in Haarkur oder als Haarspülung anwendbar. Die Zusammensetzung hat einen Gehalt an bestimmten Assoziativverdickern und Treibmitteln sowie gegebenenfalls kationaktiven, haarpflegenden Stoffen und hydrophilen Silikonen.

Übliche haarkonditionierende Präparate wie Rinse-off Kuren oder Leave-on Treatments sind in der Regel auf der Basis von wässrigen Emulsionen formuliert. Wesentliche Inhaltsstoffe sind kationaktive Substanzen wie z.B. kationische Tenside, hydrophobe Substanzen wie z.B. Fettalkohole und andere Ölkomponenten, Emulgatoren, sowie weitere spezifische Wirk- und Duftstoffe. Die wichtigsten Bestandteile sind dabei kationische Tenside, Fettalkohole und Emulgatoren. Einen Überblick über den prinzipiellen Aufbau von Kurspülungen und Haarkuren gibt Schrader, 'Grundlagen und Rezepturen der Kosmetika', 2. Auflage, 1989, Seiten 728 bis 737. Hauptaufgaben der Konditioniermittel sind die Verbesserung der Frisierbarkeit, der Kämmbarkeit, des Glanzes und des Griffs des behandelten Haares. Die behandelten Haare fühlen sich häufig etwas schwerer und belasteter an, was nicht immer erwünscht ist. Die herkömmlichen O/W-Haarkuremulsionen sind darüberhinaus normalerweise milchig-weiß und undurchsichtig. Wünschenswert sind Produkte, welche in einer optisch ansprechenderen Form vorliegen und klar, durchsichtig oder zumindest durchscheinend sind. Verschiedene Formen klarer Haarkurmittel sind zwar bekannt und beispielsweise beschrieben in E. Flick, "Cosmetic and Toiletry Formulations", Second Edition Volume 2, Seiten 373 ff. Diese klaren Haarkurmittel sind auf Basis von verdickend wirkenden Polymeren wie z.B. Cellulosederivaten (Handelsnamen Natrosol®, Methocel®), hochmolekularen Chitosanderivaten (Handelsname Kytamer® PC), komplexen Polysacchariden (Handelsnamen Karaya Gum, Tragant, Jaguar® Typen, Keltrol® Typen) und Acrylsäurepolymerisaten hergestellt. Alle diese beschriebenen klaren Haarkurmittel haben den großen Nachteil, dass die Pflegewirkung so schwach ist, dass sie bei weitem nicht diejenige eines klassischen Haarkurmittels auf Basis von Mischungen von Fettalkoholen und quatemären Tensiden erreicht. Diese, nach dem Stand der Technik bekannten klaren Haarkurmittel verkaufen sich deshalb auf dem Markt deutlich schlechter als die Standardkuren.

Es wurde gefunden, dass sich bei Verwendung von nichtionischen, amphiphilen Assoziatiwerdickem Mittel herstellen lassen, welche die typischen, an ein Haarkonditioniermittel zu stellenden Anforderungen hinsichtlich Haarkonditionierung erfüllen, gleichzeitig in einer optisch ansprechenden Form vorliegen können und dem Haar einen weniger schweren und belasteten Eindruck verleihen wie nach einer Behandlung mit einem herkömmlichen Kurmittel. Es hat sich aber gezeigt, dass bei derartigen Mitteln das Aufemulgieren während der Einarbeitung in das Haar noch nicht ganz zufriedenstellend ist. Das Produkt fühlt sich nicht gehaltvoll genug an, d.h. die Emulsionsmenge ist zu gering.

Es wurde nun gefunden, daß diese Nachteile behoben werden durch eine Zusammensetzung für ein Haarbehandlungsmittel auf Basis einer Kombination von Assoziatiwerdickem mit für Schaumaerosole üblichen Treibmitteln. Gegenstand der Erfindung ist daher eine Zusamamensetzung für ein Haarbehandlungsmittel mit einem Gehalt an
(A) mindestens einem nichtionischen, amphiphilen Assoziativverdicker in einer geeigneten kosmetischen Basis, wobei der Assoziativverdicker ausgewählt ist aus den Reaktionsprodukten der säurekatalysierten Polykondensation von mindestens zweifach funktionellen Aminoplastmonomeren und mindestens zweifach funktionellen Alkylenpolyethem sowie einfach funktionellen Verbindungen mit hydrophoben Gruppen und
(B) mindestens einem Treibmittel.

Besondere Ausführungsformen der vorliegenden Erfindung sind den ablängigen Ansprüchen zu entnehmen.

Vorzugsweise enthält die erfindungsgemäße Zusammensetzung zusätzlich mindestens einen haarpflegenden Stoff (C), welcher mindestens eine kationaktive Gruppe enthält und/oder mindestens eine haarpflegende Silikonverbindung (D), welche mindestens eine hydrophile Gruppe enthält. Es zeigte sich, dass die erfindungsgemäße Kombination auch ohne Tensidzusatz bereits ein hervorragendes Schaumverhalten zeigt und die Kombination deshalb insbesondere für die Herstellung von Schaumaerosolprodukten geeignet ist.

Der Assoziatiwerdicker (A) ist in der erfindungsgemäßen Zusammensetzung vorzugsweise in einer Menge von 0,1 bis 5 Gew.%; besonders bevorzugt von 0,1 bis 2 Gew.% enthalten. Das Treibmittel ist vorzugsweise in einer Menge von 0,05 bis 40 Gew.%, besonders bevorzugt von 0,2 bis 10 Gew.% enthalten. Der kationaktive Stoff (C) ist in der erfindungsgemäßen Zusammensetzung vorzugsweise in einer Menge von 0,01 bis 10, besonders bevorzugt von 0,1 bis 5 Gew.% und die Silikonverbindung (D) in einer Menge von vorzugsweise 0,01 bis 10 Gew.%, besonders bevorzugt von 0,1 bis 5 Gew.% enthalten.

Ein mit der erfindungsgemäßen Zusammensetzung hergestelltes Haarbehandlungsmittel erfüllt die an ein Haarkonditioniermittel hinsichtlich Konditionierwirkung zu stellenden Anforderungen in bester Weise und zeigt ein verbessertes Aufemulgieren bei der Anwendung. Das Haar ist nach der Behandlung sowohl im feuchten als auch im trockenen Zustand merkbar glatter und die Nasskämmbarkeit ist merkbar verbessert. Überraschenderweise wurde gefunden, dass das Verdickungsmittel erlaubt, kationische Stoffe und die genannten Silikonverbindungen einzuarbeiten, ohne dabei negative Begleiteigenschaften des Verdickungsmittels zu fühlen. Die fachlichen Eigenschaften des erfindungsgemäßen Mittels übertreffen sogar noch die Eigenschaften einer konventionellen Haarkur auf Basis einer wässrigen Emulsion von Fettalkoholen und quatemären Tensiden. Salon Tests im Halbseitenvergleich bestätigen dem erfindungsgemäßen Mittel eine bessere Kämmbarkeit und ein natürlicheres Anfühlen der Haare. Der meistens zu beobachtende negative stumpfe Griff der Haare von Fettalkohol/Kationtensid Mischungen ist mit dem erfindungsgemäßen Mittel praktisch eliminiert. Die Haare fühlen sich leichter und unbelasteter an. Darüberhinaus ermöglicht die erfindungsgemäße Kombination in Form eines Schaum-Aerosols, dass das Mittel in einer optisch ansprechenden, klaren Formulierung konfektioniert werden kann, was wiederum die vorteilhafte Abpackung in einem transparenten Behälter, beispielsweise aus Glas oder durchsichtigem Kunststoff, z.B. Polyethylen, Polypropylen oder Polyethylenterephtalat ermöglicht. Die Applikationsform als Schaum-Aerosol ist besonders vorteilhaft zur Haarbehandlung. Das Mittel ohne Treibmittel abgefüllt fühlt sich bei der Einarbeitung in das Haar weniger gehaltvoll an, es hat eine zu geringe Emulsionsmenge. Tests mit geschulten Friseuren zeigten, dass die Applikationsform als Schaumaerosol eindeutig präferiert wird.

Der nichtionische, amphiphile Assoziatiwerdicker (A) ist ein Polymer, welches sowohl hydrophile als auch hydrophobe Gruppen enthält. Assoziatiwerdicker sind wasserlösliche Polymere und haben tensidartige hydrophobe Bestandteile, welche in der Lage sind, sich in einem hydrophilen, insbesondere wässrigen Medium sowohl mit sich selbst als auch mit anderen hydrophoben Stoffen zu assoziieren, d.h. in Wechselwirkung zu treten. Durch das daraus resultierende assoziative Netzwerk wird das Medium verdickt oder geliert. Typischerweise werden Assoziativverdicker hergestellt durch Polymerisation von Polyethylenoxid-Prepolymeren und mindestens zweifach funktionellen, polykondensierbaren Stoffen wie z.B. Isocyanaten, wobei Mono- oder Diole mit großen Aryl-, Alkyloder Aryl/Alkyl-Gruppen eingebaut werden, um die hydrophobe Modifikation bereitzustellen. Bevorzugte Assoziativverdicker sind daher hydrophob modifizierte Polyalkylenglykole. Hierbei wird der hydrophile Bestandteil durch Polyoxyalkyleneinheiten, vorzugwseise Polyoxyethylen- aber auch Polyoxypropyleneinheiten oder deren Gemisch gebildet. Der hydrophobe Bestandteil wird vorzugsweise aus Kohlenwasserstoffgruppen, z.B langkettigen Alkylgruppen, Alkylaryl- oder Arylalkylgruppen gebildet.

Besonders bevorzugte Assoziativverdicker sind hydrophob modifizierte Aminoplast-Polyether Copolymere. Bezüglich deren Struktur und Herstellung wird auf die WO 96/40815 verwiesen. In der WO 96/40815 werden wasserdispergierbare oder wasserlösliche Copolymere beschrieben, welche die Reaktionsprodukte sind einer säurekatalysierten Polykondensation von mindestens zweifach funktionellen Aminoplastmonomeren und mindestens zweifach funktionellen Alkylenpolyethern sowie einfach funktionellen Verbindungen mit hydrophoben Gruppen. Geeignete Aminoplaste sind der Figur 1 der WO 96/40815 zu entnehmen. Besonders bevorzugt sind die Glycolurilderivate der Formel X der WO 96/40815. Geeignete Alkylenpolyether sind der Figur 2 der WO 96/40815 zu entnehmen. Bevorzugte Alkylenpolyether sind Polyethylenoxiddiole. Diese können einen Ethoxylierungsgrad von 20 bis 500, vorzugsweise 50 bis 350, besonders bevorzugt von 100 bis 250 haben. Geeignete einfach funktionelle Verbindungen mit hydrophoben Gruppen sind diejenigen der Formel XIV der WO 96/40815.

Erfindungsgemäß geeignete Assoziatiwerdicker sind ausgewählt aus Polymeren der allgemeinen Formel (I) 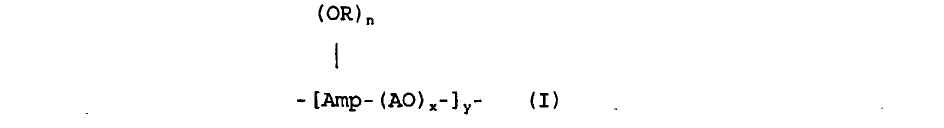 wobei Amp ein Aminoplastmonomer oder den Rest eines Aminoplastoligomers oder -polymers bedeutet, AO für eine Alkylenoxidgruppe steht, R für Wasserstoff, C1-C4-Alkyl oder C1-C4-Acyl steht und x und y Zahlen größer 1 sind.

Besonders bevorzugt sind die Reaktionsprodukte der säurekatalysierten Polykondensation von (a) Glykolurilen der allgemeinen Formel (II), 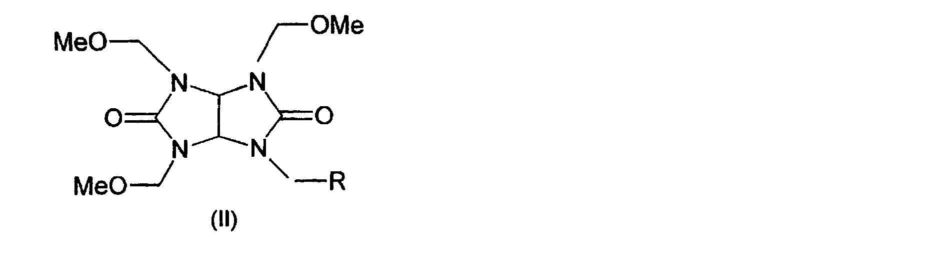 wobei R für H oder vorzugsweise für OMe steht mit (b) Polyethylenoxiddiolen eines Ethoxylierungsgrades von 20 bis 500, vorzugsweise 50 bis 350, besonders bevorzugt von 100 bis 250 sowie (c) eines gegebenenfalls ethoxylierten hydrophoben Alkohols, Alkylphenols, Thiols, Carboxamids, Carbamats oder einer hydrophoben Carbonsäure, wie sie auf den Seiten 17 bis 19 der WO 96/40815 beschrieben sind. Besonders bevorzugtes Glykoluril ist 1,3,4,6-Tetramethoxymethylglycoluril.

Geeignete Assoziatiwerdicker sind solche mit den INCI-Bezeichnungen Polyether-1, PEG-180/Octoxynol-40/TMMG Copolymer und PEG-180/Laureth-50/TMMG Copolymer und werden von der Firma Süd-Chemie vertrieben unter den Handelsbezeichnungen Pure-Thix® HH, L und M.

Zu verwendende Treibmittel (B) sind beispielsweise niedere Alkane, wie z.B. n-Butan, i-Butan und Propan, oder auch deren Gemische sowie Dimethylether oder Fluorkohlenwasserstoffe wie F152a (1,1- Difluorethan) oder F134 (Tetrafluorethan) sowie femer bei den in Betracht kommenden Drücken gasförmig vorliegende Treibmittel, wie beispielsweise N2, N2O und CO2 sowie Gemische der vorstehend genannten Treibmittel. Bevorzugt sind n-Butan, i-Butan und Propan insbesondere Propan/Butan-Gemische und Dimethylether sowie deren Gemische.

Der kationaktive Stoff (C) ist eine Substanz, die auf Grund von kationischen oder kationisierbaren Gruppen, insbesondere primären, sekundären, tertiären oder quatemären Amingruppen eine Substantivität zu menschlichem Haar aufweist. Geeignete kationaktive Stoffe sind ausgewählt aus kationischen Tensiden, betainischen Tensiden, amphoteren Tensiden, kationaktiven Polymeren mit kationischen oder kationisierbaren Gruppen, kationisch derivatisierten Proteinen, kationisch derivatisierten Proteinhydrolysaten und Betain.

Geeignete kationaktive Tenside sind Tenside, welche eine quatemäre Ammoniumgruppe enthalten. Dabei kann es sich um kationische oder um amphotere, betainische Tenside handeln. Besonders bevorzugt als kationaktiver Stoff (C) sind kationische Tenside. Geeignete kationische Tenside enthalten Aminogruppen oder quatemisierte hydrophile Ammoniumgruppen, welche in Lösung eine positive Ladung tragen und durch die allgemeine Formel (III) dargestellt werden können,

N⁽⁺⁾R¹R²R³R⁴ X⁽⁻⁾ (III)

wobei R1 bis R4 unabhängig voneinander aliphatische Gruppen, aromatische Gruppen, Alkoxygruppen, Polyoxyalkylengruppen, Alkylamidogruppen, Hydroxyalkylgruppen, Arylgruppen oder Alkarylgruppen mit 1 bis 22 C-Atomen bedeuten und X⁽⁻⁾ ein Anion darstellt, beispielsweise ein Halogen, Acetat, Phosphat, Nitrat oder Alkylsulfat, vorzugsweise ein Chlorid. Die aliphatischen Gruppen können zusätzlich zu den Kohlenstoffatomen und den Wasserstoffatomen auch Querverbindungen oder andere Gruppen wie beispielsweise Hydroxygruppen oder weitere Aminogruppen enthalten.

Beispiele für geeignete kationische Tenside sind die Chloride oder Bromide von Alkyldimethylbenzylammoniumsalzen, Alkyltrimethylammoniumsalze, beispielsweise Cetyltrimethylammoniumchlorid oder -bromid, Tetradecyltrimethylammoniumchlorid oder -bromid, Alkyldimethylhydroxyethylammoniumchloride oder-bromide, die Dialkyldimethylammoniumchloride oder-bromide, Alkylpyridiniumsalze, beispielsweise Lauryl- oder Cetylpyridiniumchlorid, Alkylamidoethyltrimethylammoniumethersulfate sowie Verbindungen mit kationischem Charakter wie Aminoxide, beispielsweise Alkylmethylaminoxide oder Alkylaminoethyldimethylaminoxide. Besonders bevorzugt sind Cetyltrimethylammoniumchlorid, das beispielsweise in Form einer 26prozentigen wässrigen Lösung unter der Handelsbezeichnung Dehyquart® A von der Firma Henkel KGaA, Düsseldorf/Deutschland und unter der Handelsbezeichnung Genamin® CTAC von der Firma Hoechst AG, Frankfurt/Deutschland sowie in Form einer 50prozentigen Lösung in Isopropanol unter der Handelsbezeichnung Arquad® 16-50 von der Firma Akzo Chemicals GmbH, Düren/Deutschland vertrieben wird.

Geeignete kationische Tenside sind auch die sogenannten Esterquats. Als Esterquats werden im allgemeinen die quaternisierten Fettsäuretriethanolaminestersalze bezeichnet. Esterquats sind beispielsweise bekannt aus der WO 91/01295. Geeignet sind Esterquats der allgemeinen Formel (VIII)

R¹CO-(OCH₂CH₂)ₓ-N⁽⁺⁾R³R⁴-(CH₂CH₂)_{y}-R² X⁽⁻⁾ (VIII)

Wobei R¹CO eine gegebenenfalls hydroxysubstiuierte C6- bis C22-Acylgruppe bedeutet, R² Wasserstoff oder eine R¹CO-Gruppe bedeutet, R³ eine C1- bis C4-Alkylgruppe oder die Gruppe (CH₂CH₂O)_{z}-H bedeutet, R⁴ eine C1- bis C4-Alkylgruppe oder die Gruppe (CH₂CH₂O)_{q}-R² bedeutet, X⁽⁻⁾ ein geeignetes Anion, beispielsweise Halogen, Alkylsulfat oder Alkylphosphat bedeutet und x, y, z und q für Zahlen von 1 bis 12 stehen. Besonders bevorzugt sind Esterquats, in denen R¹CO für eine C12- bis C20-Acylgruppe, R² für eine R¹CO-Gruppe, R³ für CH₂CH₂OH, R⁴ für Methyl und X⁽⁻⁾ für Methylsulfat steht und x und y die Zahl 1 bedeuten. Derartige Verbindungen sind unter den Handelsbezeichnungen Dehyquart® L, Dehyquart® F, Schercoquat® und Tetranyl® im Handel erhältlich.

Geeignete amphotere Tenside sind Derivate aliphatischer quaternärer Ammonium-, Phosphonium- und Sulfoniumverbindungen der Formel (IV) 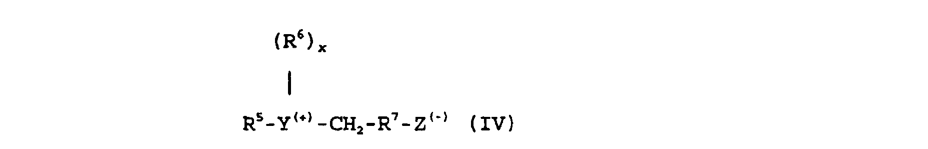 wobei R5 eine geradkettige oder verzweigtkettige Alkyl-, Alkenyl- oder Hydroxyalkylgruppe mit 8 bis 18 Kohlenstoffatomen und 0 bis etwa 10 Ethylenoxideinheiten und 0 bis 1 Glycerineinheiten darstellt; Y eine N-, P- oder S-haltige Gruppe ist; R6 eine Alkyl- oder Monohydroxyalkylgruppe mit 1 bis 3 Kohlenstoffatomen ist; x gleich 1 ist, falls Y ein Schwefelatom ist und x gleich 2 ist, wenn Y ein Stickstoffatom oder ein Phosphoratom ist; R7 eine Alkylen- oder Hydroxyalkylengruppe mit 1 bis 4 Kohlenstoffatomen ist und Z⁽⁻⁾ eine Carboxylat-, Sulfat-, Phosphonat- oder Phosphatgruppe darstellt.

Andere amphotere Tenside wie Betaine sind ebenso geeignet für das erfindungsgemäße Haarbehandlungsmittel. Beispiele für Betaine umfassen C8- bis C18-Alkylbetaine wie Cocodimethylcarboxymethylbetain, Lauryldimethylcarboxymethylbetain, Lauryldimethylalphacarboxyethylbetain, Cetyldimethylcarboxymethylbetain, Oleyldimethyl-gamma-carboxypropylbetain und Lauryl-bis(2-hydroxypropyl)alphacarboxyethylbetain; C8- bis C18-Sulfobetaine wie Cocodimethylsulfopropylbetain, Stearyldimethylsulfopropylbetain, Lauryldimethylsulfoethylbetain, Laurylbis-(2-hydroxyethyl)sulfopropylbetain; die Carboxylderivate des Imidazols, die C8- bis C18-Alkyldimethylammoniumacetate, die C8- bis C18-Alkyldimethylcarbonylmethylammoniumsalze sowie die C8- bis C18-Fettsäurealkylamidobetaine wie beispielsweise das Kokosfettsäureamidopropylbetain, welches beispielsweise in Form einer 30%igen wäßrigen Lösung unter der Handelsbezeichnung Tego® Betain L7 von der Firma Goldschmidt AG vertrieben wird und das N-Kokosfettsäureamidoethyl-N-[2-(carboxymethoxy)ethyl]-glycerin (CTFA-Name: Cocoamphocarboxyglycinate), welches zum Beispiel in Form einer 50%igen wässrigen Lösung unter der Handelsbezeichnung Miranol® C2M von der Firma Miranol Chemical Co. Inc. vertrieben wird.

Bei den geeigneten kationaktiven Polymeren handelt es sich vorzugsweise um haarfestigende oder um haarkonditionierende Polymere. Geeignete Polymere der Komponente (C) enthalten vorzugsweise quatemäre Amingruppen. Die kationischen Polymere können Homo- oder Copolymere sein, wobei die quatemären Stickstoffgruppen entweder in der Polymerkette oder vorzugsweise als Substituent an einem oder mehreren der Monomeren enthalten sind. Die Ammoniumgruppen enthaltenden Monomere können mit nicht kationischen Monomeren copolymerisiert sein. Geeignete kationische Monomere sind ungesättigte, radikalisch polymerisierbare Verbindungen, welche mindestens eine kationische Gruppe tragen, insbesondere ammoniumsubstituierte Vinylmonomere wie zum Beispiel Trialkylmethacryloxyalkylammonium, Trialkylacryloxyalkylammonium, Dialkyldiallylammonium und quatemäre Vinylammoniummonomere mit cyclischen, kationische Stickstoffe enthaltenden Gruppen wie Pyridinium, Imidazolium oder quatemäre Pyrrolidone, z.B. Alkylvinylimidazolium, Alkylvinylpyridinium, oder Alkylvinylpyrrolidon Salze. Die Alkylgruppen dieser Monomere sind vorzugsweise niedere Alkylgruppen wie zum Beispiel C1- bis C7-Alkylgruppen, besonders bevorzugt C1- bis C3-Alkylgruppen.

Die Ammoniumgruppen enthaltenden Monomere können mit nicht kationischen Monomeren copolymerisiert sein. Geeignete Comonomere sind beispielsweise Acrylamid, Methacrylamid, Alkyl- und Dialkylacrylamid, Alkyl- und Dialkylmethacrylamid, Alkylacrylat, Alkylmethacrylat, Vinylcaprolacton, Vinylcaprolactam, Vinylpyrrolidon, Vinylester, z.B. Vinylacetat, Vinylalkohol, Propylenglykol oder Ethylenglykol, wobei die Alkylgruppen dieser Monomere vorzugsweise C1- bis C7-Alkylgruppen, besonders bevorzugt C1- bis C3-Alkylgruppen sind.

Geeignete Polymere mit quaternären Amingruppen sind beispielsweise die im CTFA Cosmetic Ingredient Dictionary unter den Bezeichnungen Polyquatemium beschriebenen Polymere wie Methylvinylimidazoliumchlorid/Vinylpyrrolidon Copolymer (Polyquatemium-16) oder quatemisiertes Vinylpyrrolidon/Dimethylaminoethylmethacrylat Copolymer (Polyquaternium-11).

Von den kationischen Polymeren, die in dem erfindungsgemäßen Mittel enthalten sein können, ist zum Beispiel Vinylpyrrolidon/Dimethylaminoethylmethacrylatmethosulfat Copolymer, das unter den Handelsbezeichnungen Gafquat® 755 N und Gafquat® 734 von der Firma Gaf Co., USA vertrieben wird und von denen das Gafquat® 734 besonders bevorzugt ist, geeignet. Weitere kationische Polymere sind beispielsweise das von der Firma BASF, Deutschland unter dem Handelsnamen LUVIQUAT® HM 550 vertriebene Copolymer aus Polyvinylpyrrolidon und Imidazoliminmethochlorid, das von der Firma Calgon/USA unter dem Handelsnamen Merquat® Plus 3300 vertriebene Terpolymer aus Dimethyldiallylammoniumchlorid, Natriumacrylat und Acrylamid, das von der Firma ISP/USA unter dem Handelsnamen Gaffix® VC 713 vertriebene Terpolymer aus Vinylpyrrolidon, Dimethylaminoethylmethacrylat und Vinylcaprolactam und das von der Firma Gaf unter dem Handelsnamen Gafquat® HS 100 vertriebene Vinylpyrrolidon/Methacrylamidopropyltrimethylammoniumchlorid Copolymer.

Geeignete kationische Polymere, die von natürlichen Polymeren abgeleitet sind, sind kationische Derivate von Polysacchariden, beispielsweise kationische Derivate von Cellulose, Stärke oder Guar. Geeignet sind weiterhin Chitosan und Chitosanderivate. Kationische Polysaccharide haben die allgemeine Formel (V)

G-O-B-N⁺R^{a}R^{b}R^{c} X⁻ (V)

G ist ein Anhydroglucoserest, beispielsweise Stärke- oder Celluloseanhydroglucose; B ist eine divalente Verbindungsgruppe, beispielsweise Alkylen, Oxyalkylen, Polyoxyalkylen oder Hydroxyalkylen; R^{a}, R^{b} und R^{c} sind unabhängig voneinander Alkyl, Aryl, Alkylaryl, Arylalkyl, Alkoxyalkyl oder Alkoxyaryl mit jeweils bis zu 18 C-Atomen, wobei die Gesamtzahl der C-Atome in R^{a}, R^{b} und R^{c} vorzugsweise maximal 20 ist;
X⁽⁻⁾ ist ein übliches Gegenanion, hat die gleiche Bedeutung wie bei Formel (I) und ist vorzugsweise Chlorid. Eine kationische Cellulose wird unter der Bezeichnung Polymer JR von Amerchol vertrieben und hat die INCI-Bezeichnung Polyquaternium-10. Eine weitere kationische Cellulose trägt die INCI-Bezeichnung Polyquaternium-24 und wird unter dem Handelsnamen Polymer LM-200 von Amerchol vertrieben. Ein geeignetes kationisches Guarderivat wird unter der Handelsbezeichnung Jaguar® R vertrieben und hat die INCI-Bezeichnung Guar Hydroxypropyltrimonium Chloride.

Besonders bevorzugte kationaktive Stoffe sind Chitosan, Chitosansalze und Chitosan-Derivate. Bei den erfindungsgemäß einzusetzenden Chitosanen handelt es sich um vollständig oder partiell deacetylierte Chitine. Zur Herstellung von Chitosan geht man vorzugsweise von dem in den Schalenresten von Krustentieren enthaltenem Chitin aus, welches als billiger und natürlicher Rohstoff in großen Mengen zur Verfügung steht. Das Molekulargewicht des Chitosans kann über ein breites Spektrum verteilt sein, beispielsweise von 20.000 bis ca. 5 Millionen g/mol. Geeignet ist beispielsweise ein niedermolekulares Chitosan mit einem Molekulargewicht von 30.000 bis 70.000 g/mol. Vorzugsweise liegt das Molekulargewicht jedoch über 100.000 g/mol, besonders bevorzugt von 200.000 bis 700.000 g/mol. Der Deacetylierungsgrad beträgt vorzugsweise 10 bis 99%, besonders bevorzugt 60 bis 99%.

Ein geeignetes Chitosan wird beispielsweise von der Firma Kyowa Oil&Fat, Japan, unter dem Handelsnamen Flonac® vertrieben. Es hat ein Molekulargewicht von 300.000 bis 700.000 g/mol und ist zu 70 bis 80% entacetyliert. Ein bevorzugtes Chitosansalz ist Chitosoniumpyrrolidoncarboxylat, welches beispielsweise unter der Bezeichnung Kytamer PC von der Firma Amerchol, USA, vertrieben wird. Das enthaltene Chitosan hat ein Molekulargewicht von ca. 200.000 bis 300.000 g/mol und ist zu 70 bis 85% entacetyliert. Als Chitosanderivate kommen quatemierte, alkylierte oder hydroxyalkylierte Derivate, beispielsweise Hydroxyethyl- oder Hydroxybutylchitosan in Betracht.

Die Chitosane oder Chitosanderivate liegen vorzugsweise in neutralisierter oder partiell neutralisierter Form vor. Der Neutralisationsgrad für das Chitosan oder das Chitosanderivat liegt vorzugsweise bei mindestens 50%, besonders bevorzugt zwischen 70 und 100%, bezogen auf die Anzahl der freien Basengruppen. Als Neutralisationsmittel können prinzipiell alle kosmetisch verträglichen anorganischen oder organischen Säuren verwendet werden wie beispielsweise Ameisensäure, Weinsäure, Äpfelsäure, Milchsäure, Zitronensäure, Pyrrolidoncarbonsäure, Salzsäure u.a., von denen die Pyrrolidoncarbonsäure besonders bevorzugt ist.

Weitere geeignete kationaktive, haarpflegende Verbindungen sind kationisch modifizierte Proteinderivate oder kationisch modifizierte Proteinhydrolysate und sind beispielsweise bekannt unter den INCI-Bezeichnungen Lauryldimonium Hydroxypropyl Hydrolyzed Wheat Protein, Lauryldimonium Hydroxypropyl Hydrolyzed Casein, Lauryldimonium Hydroxypropyl Hydrolyzed Collagen, Lauryldimonium Hydroxypropyl Hydrolyzed Keratin, Lauryldimonium Hydroxypropyl Hydrolyzed Silk, Lauryldimonium Hydroxypropyl Hydrolyzed Soy Protein oder Hydroxypropyltrimonium Hydrolyzed Wheat, Hydroxypropyltrimonium Hydrolyzed Casein, Hydroxypropyltrimonium Hydrolyzed Collagen, Hydroxypropyltrimonium Hydrolyzed Keratin, Hydroxypropyltrimonium Hydrolyzed Rice Bran Protein, Hydroxypropyltrimonium Hydrolyzed Silk, Hydroxypropyltrimonium Hydrolyzed Soy Protein, Hydroxypropyltrimonium Hydrolyzed Vegetable Protein.

Geeignete kationisch derivatisierte Proteinhydrolysate sind Substanzmischungen, die beispielsweise durch Umsetzung von alkalisch, sauer oder enzymatisch hydrolysierten Proteinen mit Glycidyltrialkylammoniumsalzen oder 3-Halo-2-hydroxypropyltrialkylammoniumsalzen erhalten werden können. Proteine, die als Ausgangsstoffe für die Proteinhydrolysate dienen, können sowohl pflanzlicher als auch tierischer Herkunft sein. Übliche Ausgangsstoffe sind beispielsweise Keratin, Collagen, Elastin, Sojaprotein, Reisprotein, Milchprotein, Weizenprotein, Seidenprotein oder Mandelprotein. Durch die Hydrolyse entstehen Stoffmischungen mit Molmassen im Bereich von ca. 100 bis ca. 50.000. Übliche mittlere Molmassen liegen im Bereich von etwa 500 bis etwa 1000. Vorteilhafterweise enthalten die kationisch derivatisierten Proteinhydrolysate eine oder zwei lange C8- bis C22-Alkylketten und entsprechend zwei oder eine kurze C1- bis C4-Alkylketten. Verbindungen, die eine lange Alkylkette enthalten, sind bevorzugt.

Die hydrophilen Gruppen der erfindungsgemäß einzusetzenden haarpflegenden Silikonverbindungen (D) sind vorzugsweise ausgewählt aus Hydroxylgruppen, primären, sekundären oder tertiären Aminogruppen, quatemären Ammoniumgruppen, Alkylenoxidgruppen, betainischen Gruppen und Thiosulfatgruppen.

Geeignet und besonders bevorzugt sind kationaktive Silikonverbindungen. Diese sind mit kationischen oder kationisierbaren Gruppen substituiert. Geeignete kationaktive Silikonverbindungen weisen entweder mindestens eine Aminogruppe oder mindestens eine Ammoniumgruppe auf. Geeignete Silikonpolymere mit Aminogruppen sind unter der INCI-Bezeichnung Amodimethicone bekannt. Hierbei handelt es sich um Polydimethylsiloxane mit Aminoalkylgruppen. Die Aminoalkylgruppen können seiten- oder endständig sein. Geeignete Aminosilikone sind solche der allgemeinen Formel (VI)

R⁸R⁹R¹⁰Si-(OSiR¹¹R¹²)x-(OSiR¹³Q)y-OSiR¹⁴R¹⁵R¹⁶ (VI)

R⁸, R⁹, R¹⁴ und R¹⁵ sind unabhängig voneinander gleich oder verschieden und bedeuten C1- bis C10-Alkyl, Phenyl, Hydroxy, Wasserstoff, C1- bis C10-Alkoxy oder Acetoxy, vorzugsweise C1-C4-Alkyl, besonders bevorzugt Methyl; R¹⁰ und
R¹⁶ sind unabhängig voneinander gleich oder verschieden und bedeuten -(CH₂)ₐ-NH₂ mit a gleich 1 bis 6, C1- bis C10-Alkyl, Phenyl, Hydroxy, Wasserstoff, C1- bis C10-Alkoxy oder Acetoxy, vorzugsweise C1-C4-Alkyl, besonders bevorzugt Methyl; R¹¹, R¹² und R¹³ sind unabhängig voneinander gleich oder verschieden und bedeuten Wasserstoff, C1- bis C20-Kohlenwasserstoff, welcher O- und N-Atome enthalten kann, vorzugsweise C1- bis C10-Alkyl oder Phenyl, besonders bevorzugt C1- bis C4-Alkyl, insbesondere Methyl; Q bedeutet -A-NR¹⁷R¹⁸, oder-A-N⁺R¹⁷R¹⁸R¹⁹ wobei A für eine divalente C1- bis C20-Alkylenverbindungsgruppe steht, welche auch O- und N-Atome sowie OH-Gruppen enthalten kann, und R¹⁷, R¹⁸ und R¹⁹ unabhängig voneinander gleich oder verschieden sind und Wasserstoff, C1- bis C22-Kohlenwasserstoff, vorzugsweise C1- bis C-4-Alkyl oder Phenyl bedeuten. Bevorzugte Reste für Q sind -(CH₂)₃-NH₂, -(CH₂)₃NHCH₂CH₂NH₂, -(CH₂)₃OCH₂CHOHCH₂NH₂ und -(CH₂)₃N(CH₂CH₂OH)₂, -(CH₂)₃-NH₃⁺ und -(CH₂)₃OCH₂CHOHCH₂N⁺(CH₃)₂R²⁰, wobei R²⁰ ein C1- bis C22-Alkylrest ist, der auch OH-Gruppen aufweisen kann; x bedeutet eine Zahl zwischen 1 und 10.000, vorzugsweise zwischen 1 und 1.000; y bedeutet eine Zahl zwischen 1 und 500, vorzugsweise zwischen 1 und 50.

Das Molekulargewicht der Aminosilikone liegt vorzugsweise zwischen 500 und 100.000. Der Aminanteil (meq/g) liegt vorzugsweise im Bereich von 0,05 bis 2,3, besonders bevorzugt von 0,1 bis 0,5.

Geeignete Silikonpolymere mit zwei endständigen quatemären Ammoniumgruppen sind unter der INCI-Bezeichnung Quaternium-80 bekannt. Hierbei handelt es sich um Dimethylsiloxane mit zwei endständigen Aminoalkylgruppen. Geeignete quaternäre Aminosilikone sind solche der allgemeinen Formel (VII)

R²¹R²²R²³N⁺-A-SiR⁸R⁹-(OSiR¹¹R¹²)ₙ-OSiR⁸R⁹-A-N⁺R²¹R²²R²³ 2X⁻ (VII)

A hat die gleiche Bedeutung wie oben bei Formel (VI) angegeben und ist vorzugsweise -(CH₂)₃OCH₂CHOHCH₂N⁺(CH₃)₂R²⁰, wobei R²⁰ ein C1- bis C22-Alkylrest ist, der auch OH-Gruppen aufweisen kann; R⁸, R⁹, R¹¹ und R¹² haben die gleiche Bedeutung wie oben bei Formel (VI) angegeben und sind vorzugsweise Methyl; R²¹, R²², und R²³ bedeuten unabhängig voneinander C1-bis C22-Alkylreste, welche Hydroxygruppen enthalten können und wobei vorzugsweise mindestens einer der Reste mindestens 10 C-Atome aufweist und die übrigen Reste 1 bis 4 C-Atome aufweisen; n ist eine Zahl von 0 bis 200, vorzugsweise von 10 bis 100. Derartige diquaternäre Polydimethylsiloxane werden von der Firma GOLDSCHMIDT/Deutschland unter den Handelsnamen Abil® Quat 3270, 3272 und 3274 vertrieben.

Geeignete Silikone mit Alkylenoxidgruppen sind Polydimethylsiloxane mit polyoxyalkylierten Substituenten, insbesondere Silikone, die mit Polypropylenoxid, Polyethylenoxid oder deren Gemisch modifiziert sind. Die Alkylenoxidgruppen können dabei seitenständig oder endständig sein oder es kann sich um Polydimethylsiloxan/Polyalkylenoxid Blockcopolymere handeln. Die mit Alkylenoxiden modifizierten Siloxane werden auch als Dimethylsiloxanglykolcopolymere oder als Dimethicon Copolyole bezeichnet. Geeignete Silikone mit Hydroxylgruppen sind Dimethiconole. Hierbei handelt es sich um Polydimethylsiloxane mit Hydroxylendgruppen. Geeignete Silikone mit Thiosulfatgruppen sind bekannt unter der INCI-Bezeichnung Dimethicone/Sodium PG-Propyldimethicone Thiosulfate Copolymer.

Das erfindungsgemäße Mittel wird bevorzugt in einem wässrigen oder in einem wässrig-alkoholischen Milieu konfektioniert und zeichnet sich besonders durch seine Klarheit und Transparenz aus. Daher wird das Mittel vorteilhafterweise auch in eine optisch ansprechende Verpackung aus durchsichtigem oder durchscheinendem, druckfestem Material abgefüllt. Als Verpackungsmaterial kommen insbesondere Glas und und durchsichtige oder durchscheinende Kunststoffe wie z.B. Polyethylenterephtalat in Betracht. Als Alkohole können insbesondere die für kosmetische Zwecke üblicherweise verwendeten niederen Alkohole mit 1 bis 4 Kohlenstoffatomen wie zum Beispiel Ethanol und Isopropanol enthalten sein. Der Wassergehalt beträgt vorzugsweise von 40 bis 95, besonders bevorzugt von 60 bis 90 Gewichtsprozent. Der Alkoholgehalt beträgt vorzugsweise von 1 bis 30, besonders bevorzugt von 5 bis 20 Gewichtsprozent. Weitere, besonders bevorzugte wasserlösliche Lösungs- bzw. Feuchthaltemittel sind mehrwertige Alkohole, insbesondere solche mit 2 bis 4 Kohlenstoffatomen wie beispielsweise Glycerin, Ethylenglykol oder Propylenglykol in einer Menge von 0,1 bis 10 Gew.%, vorzugsweise von 0,5 bis 5 Gew.%. Besonders bevorzugt sind rein wässrige Formulierungen.

In einer bevorzugten Ausführungsform enthält das erfindungsgemäße Mittel zusätzlich mindestens ein nichtionisches Tensid. Geeignete nichtionische Tenside sind beispielsweise die im "International Cosmetic Ingredient Dictionary and Handbook", 7. Auflage, Band 2 im Abschnitt "Surfactants - Emulsifying Agents" aufgeführten nichtionischen Emulgatoren. Geeignete nichtionische Tenside sind vorzugsweise ausgewählt aus ethoxylierten Fettsäuren mit 10 bis 26 Kohlenstoffatomen, ethoxylierten ein- oder mehrwertigen Alkoholen mit 1 bis 6 Kohlenstoffatomen, ethoxylierten Fettalkoholen mit 10 bis 26 Kohlenstoffatomen, ethoxyliertem hydriertem oder nicht hydriertem Rizinusöl, Alkylpolyglucosiden, Glyceridalkoxylaten, Fettsäureglyceridpolyalkylenglykolethem oder Fettsäurepartialglyceridpolyalkylenglykolethern mit jeweils weniger als 30 Alkylenglykoleinheiten wie beispielsweise Polyethylenglykol-(7)-glycerylcocoat, Polyglykolamiden, Fettsäurezuckerestem, ethoxylierten Fettsäurezuckerestem und Partialglyceriden. Der Ethoxylierungsgrad von ethoxylierten Tensiden beträgt üblicherweise von 1 bis 400, vorzugsweise 2 bis 200, besonders bevorzugt 3 bis 25.

In einer bevorzugten Ausführungsform sind in dem erfindungsgemäßen Mittel nur solche Tenside und Emulgatoren enthalten, welche wasserlöslich sind, d.h. solche Tenside, die bei einem Gehalt von 1 Gewichtsprozent in Wasser bei 20°C klar löslich sind.

Bevorzugte nichtionische Tenside sind insbesondere Fettalkoholethoxylate. Geeignet sind beispielsweise Alkohole mit 10 bis 18, vorzugsweise 10 bis 16 C-Atomen und einem Ethoxylierungsgrad von vorzugsweise 2 bis 200, besonders bevorzugt von 3 bis 25. Die zusätzlichen nichtionischen Tenside werden in einer Menge von vorzugsweise 0,01 bis 5 Gew.% eingesetzt.

In einer weiteren bevorzugten Ausführungsform enthält das erfindungsgemäße Mittel zusätzlich mindestens ein filmbildendes, haarfestigendes, synthetisches oder natürliches Polymer. Dieses zusätzliche Polymer kann nichtionischen, anionischen oder amphoteren Charakter haben und wird bevorzugt in einer Menge von 0,5 bis 10 Gew. % eingesetzt. Unter filmbildenden, haarfestigenden Polymeren werden solche Polymere verstanden, welche in 0.1 bis 5 %iger wässriger, alkoholischer oder wässrig-alkoholischer Lösung angewandt in der Lage sind, auf dem Haar einen Polymerfilm abzuscheiden und auf diese Weise das Haar zu festigen.

Das erfindungsgemäße Mittel kann darüber hinaus die für Haarbehandlungsmittel üblichen Zusatzbestandteile enthalten, zum Beispiel nicht festigende nichtionische Polymere, nicht festigende anionische Polymere und nicht festigende natürliche Polymere sowie deren Kombination in einer Menge von vorzugsweise 0,01 bis 10 Gew. %; Parfümöle in einer Menge von vorzugsweise 0,01 bis 5 Gew. %; Netzmittel oder Emulgatoren aus den Klassen der anionischen, kationischen, amphoteren oder nichtionogenen oberflächenaktiven Substanzen in einer Menge von vorzugsweise 0.01 bis 10 Gew. %; Feuchthaltemittel; Konservierungsmittel, bakterizide und fungizide Wirkstoffe wie zum Beispiel 2,4,4-Trichlor-2-hydroxydiphenylether, Parabene oder Methylchlorisothiazolinon, in einer Menge von 0,01 bis 1,0 Gewichtsprozent; Puffersubstanzen, wie beispielsweise Natriumcitrat oder Natriumphosphat, in einer Menge von 0,1 bis 1,0 Gewichtsprozent; Anfärbestoffe, wie zum Beispiel Fluorescein Natriumsalz, in einer Menge von etwa 0,1 bis 1,0 Gewichtsprozent; Pflegestoffe, wie zum Beispiel Pflanzen- und Kräuterextrakte, Protein- und Seidenhydrolysate, Lanolinderivate, in einer Menge von 0,1 bis 5 Gewichtsprozent; Lichtschutzmittel, Antioxidantien, Radikalfänger, Antischuppenwirkstoffe, Fettalkohole, Glanzgeber, Vitamine und rückfettende Agenzien in einer Menge von 0,01 bis 10 Gew.%.

Das erfindungsgemäße Mittel kann in einem pH-Bereich von 2,0 bis 9,5 vorliegen. Besonders bevorzugt sind schwach saure pH-Werte im Bereich zwischen 4,5 und kleiner 7, besonders bevorzugt von 5,5 bis 6,5. Liegt das erfindungsgemäße Mittel im sauren Bereich vor, so kann es organische oder anorganische Säuren enthalten wie beispielsweise Ameisensäure, Weinsäure, Äpfelsäure, Maleinsäure, Fumarsäure, Glyoxylsäure, Pyrrolidoncarbonsäure, Zitronensäure, Milchsäure, Schwefelsäure, Essigsäure, Salzsäure, Phosphorsäure u.a..

Das erfindungsgemäße Mittel wird angewendet, indem eine für den gewünschten Konditioniereffekt ausreichende Menge in oder auf dem trockenen Haar oder nach der Haarwäsche in oder auf dem nassen oder feuchten Haar verteilt wird. Die anzuwendende Menge hängt von der Haarfülle ab und beträgt typischerweise 1 bis 25 g. Bei der bevorzugten Verwendung als Rinse-Produkt wird nach einer ausreichenden Einwirkzeit von beispielsweise 1 bis 15 Minuten das Haar ausgespült. Anschließend wird das Haar gegebenenfalls durchgekämmt oder zur Frisur geformt und getrocknet. Bei einer Verwendung als Leave-in-Produkt wird das Haar nach Aufbringen des Mittels nicht ausgespült.

Die nachfolgenden Beispiele sollen den Gegenstand der Erfindung näher erläutern.

### Beispiel 1: Klare Haarkur für strapaziertes Haar

| | |
|---|---|
| 3,0 g | Arquad® 12-25 (25%ig, Lauryltrimoniumchlorid) |
| 2,0 g | Abil® 9950 (30%ig, Dimethicone Propyl PG-Betaine) |
| 1,1 g | Pure Thix® HH (Polyether-1) |
| 0,5 g | Brij® 30 (Laureth-4) |
| ad 100 g | Wasser |

Die Zusammensetzung wird abgefüllt mit 8 Gew.%, bezogen auf die Gesamtzusammensetzung an Dimethylether.

### Beispiel 2: Klare Haarspülung für dauergewelltes Haar

| | |
|---|---|
| 1,0 g | Arquad® 12-50 (50%ig, Lauryltrimoniumchlorid) |
| 0,6 g | Tegobetain® (30%ig in Wasser, Cocamidopropyl Betaine) |
| 0,8 g | Abil® Quat 3272 (50%ig in Propylenglykol, Quaternium-80, diquatemäres Silikon) |
| 0,8 g | Pure Thix® L (PEG-180/Octoxynol-40/TMMG Copolymer) |
| 0,2 g | Glyoxylsäure (1%ige Lösung) |
| ad 100 g | Wasser |

Die Zusammensetzung wird abgefüllt mit 2 Gew.% bezogen auf die Gesamtzusammensetzung an Propan/Butan sowie 6 Gew.% bezogen auf die Gesamtzusammensetzung an Dimethylether.

### Beispiel 3: Klares Leave-in-Treatment

| | |
|---|---|
| 1,0 g | Tallowtrimoniumchlorid |
| 2,0 g | Abil® S 201 (30%ig in Isopropanol/Wasser, Dimethicone/Sodium PG-Propyldimethicone Thiosulfate Copolymer) |
| 1,1 g | Pure Thix® M (PEG-180/Laureth-50/TMMG Copolymer) |
| 3,0 g | Luviquat® FC 905 (40%ig in Wasser, Polyquatemium-16) |
| ad 100 g | Wasser |

Die Zusammensetzung wird abgefüllt mit 5 Gew.%, bezogen auf die Gesamtzusammensetzung an Dimethylether.

### Beispiel 4: Klare Haarkur zum Entwirren der Haare

| | |
|---|---|
| 2,5 g | Arquad® 12-50 (50%ig, Lauryltrimoniumchlorid) |
| 1,8 g | Abil® Quat 3270 (50%ig in Propylenglykol, Quaternium-80, diquatemäres Silikon) |
| 1,3 g | Pure Thix® HH (Polyether-1) |
| 0,3 g | Brij® 30 (Laureth-4) |
| 0,2 g | Zitronensäure (1%ige Lösung) |
| ad 100 g | Wasser |

Die Zusammensetzung wird abgefüllt mit 10 Gew.%, bezogen auf die Gesamtzusammensetzung an Dimethylether.

### Beispiel 5: Klare Haarspülung - besonders mild

| | |
|---|---|
| 0,8 g | Arquad® 12-25 (25%ig, Lauryltrimoniumchlorid) |
| 1,0 g | Dow Corning® 193 (Dimethicone Copolyol) |
| 1,0 g | Pure Thix® M (PEG-180/Laureth-50/TMMG Copolymer) |
| 0,5 g | Rewoteric® AM CAS (50%ig in Wasser, Cocamidopropyl Hydroxysultaine) |
| ad 100 g | Wasser |

Die Zusammensetzung wird abgefüllt mit 6 Gew.%, bezogen auf die Gesamtzusammensetzung an Dimethylether sowie 3 Gew.%, bezogen auf die Gesamtzusammensetzung an F152a.

### Beispiel 6: Klares Leave-in-Treatment

| | |
|---|---|
| 1,0 g | Arquad® 12-50 (50%ig, Lauryltrimoniumchlorid) |
| 2,0 g | Abil® 8863 (Dimethicone Copolyol) |
| 0,9 g | Pure Thix® HH (Polyether-1) |
| 0,5 g | Luviskol® K30 (Polyvinylpyyrolidon) |
| ad 100 g | Wasser |

Die Zusammensetzung wird abgefüllt mit 5 Gew.%, bezogen auf die Gesamtzusammensetzung an Dimethylether.

## Patentansprüche

1. Zusammensetzung für ein Haarbehandlungsmittel mit einem Gehalt an
(A) mindestens einem nichtionischen, amphiphilen Assoziativverdicker in einer geeigneten kosmetischen Basis, wobei der Assoziatiwerdicker ausgewählt ist aus den Reaktionsprodukten der säurekatalysierten Polykondensation von mindestens zweifach funktionellen Aminoplastmonomeren und mindestens zweifach funktionellen Alkylenpolyethem sowie einfach funktionellen Verbindungen mit hydrophoben Gruppen und
(B) mindestens einem Treibmittel.

2. Zusammensetzung nach Anspruch 1, **dadurch gekennzeichnet, dass** die Aminoplastmonomere ausgewählt sind aus 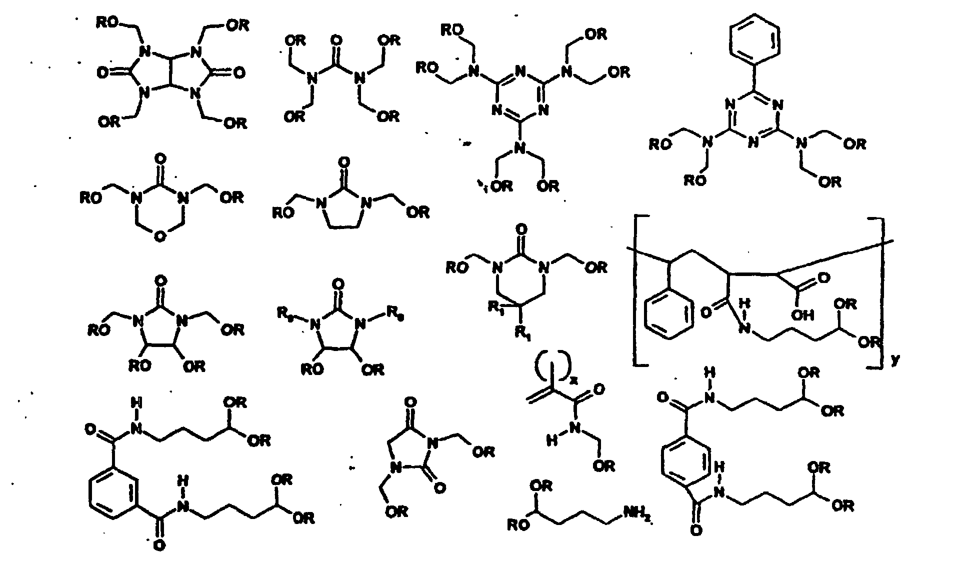 wobei R Wasserstoff, C1- bis C4-Alkyl oder C1- bis C4-Acyl ist; R₁ C1- bis C4-Alkyl ist, x 0 oder 1 ist und y mindestens 2 ist; R₀ C1 - bis C4-Alkyl, Aryl, Cycloalkyl ist.

3. Zusammensetzung nach einem der vorhergehenden Ansprüchen, **dadurch gekennzeichnet, dass** der Verdicker ausgewählt ist aus den Reaktionsprodukten der säurekatalysierten Reaktion von Glycolurilderivaten und Polyalkylenglykden und alkoxylierten Kohlenwasserstoffen.

4. Zusammensetzung nach einem der vorhergehenden Ansprüche, **dadurch gekennzeichnet, dass** der Verdicker ausgewählt ist aus den Reaktionsprodukten der säurekatalysierten Reaktion von (a) Glykolurilen der allgemeinen Formel (II), 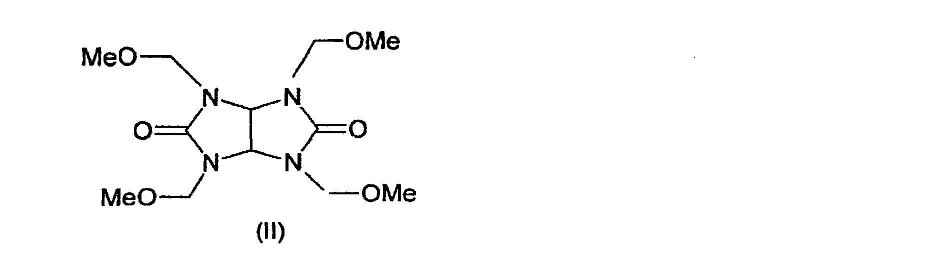 mit (b) Polyethylenoxiddiolen eines Ethoxylierungsgrades von 20 bis 500 sowie (c) eines gegebenenfalls ethoxylierten hydrophoben Alkohols, Alkylphenols, Thiols, Carboxamids, Carbamats oder einer hydrophoben Carbonsäure.

5. Zusammensetzung nach einem der vorhergehenden Ansprüche, **dadurch gekennzeichnet, dass** der Verdicker ausgewählt ist aus Polyether-1, PEG-180/Octoxynol-40/ TMMG Copolymer und PEG-180/Laureth-50/TMMG Copolymer.

6. Zusammensetzung nach einem der vorhergehenden Ansprüche, **dadurch gekennzeichnet, dass** das Treibmittel ausgewählt ist aus n-Butan, Isobutan, Propan, Dimethylether, Fluorkohlenwasserstoffen und Gemischen der genannten Treibmittel.

7. Zusammensetzung nach einem der vorhergehenden Ansprüche, **dadurch gekennzeichnet, dass** es zusätzlich mindestens einen haarpflegenden Stoff (C) enthält, welcher mindestens eine kationaktive Gruppe aufweist.

8. Zusammensetzung nach Anspruch 7, **dadurch gekennzeichnet, dass** der haarpflegende kationaktive Stoff ausgewählt ist aus kationaktiven Tensiden, Polymeren mit kationischen oder kationisierbaren Gruppen, kationisch derivatisierten Proteinen, kationisch derivatisierten Proteinhydrolysaten und Betain.

9. Zusammensetzung nach Anspruch 8, **dadurch gekennzeichnet, dass** das kationische Tensid ausgewählt ist aus Verbindungen der Formel (III)
N⁽⁺⁾R¹R²R³R⁴ X⁽⁻⁾ (III)
wobei R1 bis R4 unabhängig voneinander aliphatische Gruppen, aromatische Gruppen, Alkoxygruppen, Polyoxyalkylengruppen, Alkylamidogruppen, Hydroxyalkylgruppen, Arylgruppen oder Alkarylgruppen mit 1 bis 22 C-Atomen bedeuten und X⁽⁻⁾ ein Anion darstellt.

10. Zusammensetzung nach Anspruch 8, **dadurch gekennzeichnet, dass** das Polymer mit kationischen oder kationisierbaren Gruppen ausgewählt ist aus MethylvinylimidazoliumchloridNinylpyrrolidon Copolymeren, quatemisierten Vinylpyrrolidon/Dimethylaminoethylmethacrylat Copolymeren, kationisch derivatisierten Polysacchariden, Chitosan, Chitosansalzen und Chitosanderivaten.

11. Zusammensetzung nach einem der vorhergehenden Ansprüche, **dadurch gekennzeichnet, dass** es zusätzlich mindestens eine Silikonverbindung (D) enthält, welche mindestens eine hydrophile Gruppe aufweist.

12. Zusammensetzung nach Anspruch 11, **dadurch gekennzeichnet**, dassdie hydrophile Gruppe der Silikonverbindung ausgewählt ist aus Hydroxylgruppen, primären, sekundären oder tertiären Aminogruppen, quatemären Ammoniumgruppen, Alkylenoxidgruppen, betainischen Gruppen und Thiosulfatgruppen.

13. Zusammensetzung nach Anspruch 11 oder 12, **dadurch gekennzeichnet, dass** die Silikonverbindung mit mindestens einer hydrophilen Gruppe ausgewählt ist aus Silikonverbindungen mit kationischen Gruppen, hydroxysubstituierten Siloxanen, Siloxan/Polyoxyalkylen Copolymeren und aminosubstituierten Siloxanen.

14. Haarbehandlungsmittel mit einem Gehalt an einer Zusammensetzung nach einem der vorhergehenden Ansprüche, **dadurch gekennzeichnet, dass** es in einer optisch klaren Form vorliegt.

15. Haarbehandlungsprodukt bestehend aus einer durchsichtigen oder durchscheinenden Verpackung enthaltend eine Zusammensetzung nach einem der vorhergehenden Ansprüche.

## Claims

1. Composition for a hair-treatment agent with a content of
(A) at least one nonionic, amphiphilic associative thickener in a suitable cosmetic base, where the associative thickener is chosen from the reaction products of the acid-catalysed polycondensation of at least difunctional aminoplastic monomers and at least difunctional alkylene polyethers, and monofunctional compounds with hydrophobic groups and
(B) at least one propellant.

2. Composition according to Claim 1, **characterized in that** the aminoplastic monomers are chosen from 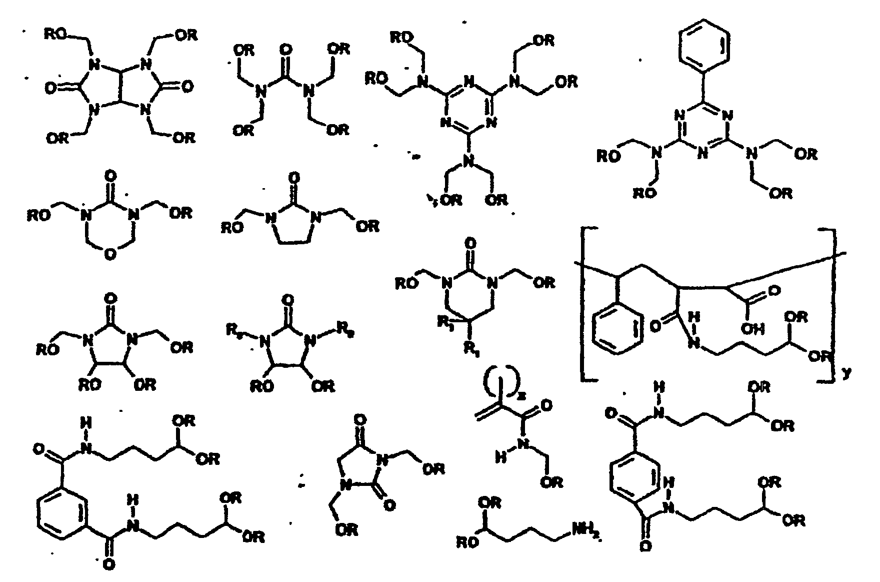 where R is hydrogen, C1-C4-alkyl or C1-C4-acyl; R₁ is C1-C4-alkyl, x is 0 or 1 and y is at least 2; R₀ is C1-C4-alkyl, aryl, cycloalkyl.

3. Composition according to one of the preceding claims, **characterized in that** the thickener is chosen from the reaction products of the acid-catalysed reaction of glycoluril derivatives and polyalkylene glycols and alkoxylated hydrocarbons.

4. Composition according to one of the preceding claims, **characterized in that** the thickener is chosen from the reaction products of the acid-catalysed reaction of (a) glycolurils of the general formula (II), 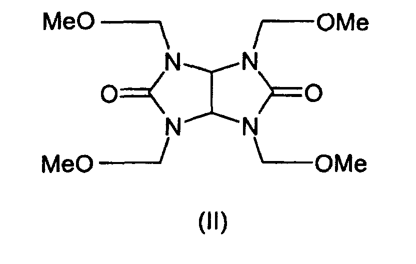 with (b) polyethylene oxide diols with a degree of ethoxylation of from 20 to 500, and (c) an optionally ethoxylated hydrophobic alcohol, alkylphenol, thiol, carboxamide, carbamate or a hydrophobic carboxylic acid.

5. Composition according to one of the preceding claims, **characterized in that** the thickener is chosen from polyether-1, PEG-180/Octoxynol-40/TMMG copolymer and PEG-180/Laureth-50/TMMG copolymer.

6. Composition according to one of the preceding claims, **characterized in that** the propellant is chosen from n-butane, isobutane, propane, dimethyl ether, fluorinated hydrocarbons and mixtures of said propellants.

7. Composition according to one of the preceding claims, **characterized in that** it additionally comprises at least one haircare substance (C) which has at least one cation-active group.

8. Composition according to Claim 7, **characterized in that** the haircare cation-active substance is chosen from cation-active surfactants, polymers with cationic or cationizable groups, cationically derivatized proteins, cationically derivatized protein hydrolysates and betaine.

9. Composition according to Claim 8, **characterized in that** the cationic surfactant is chosen from compounds of the formula (III)
N⁽⁺⁾R¹R²R³R⁴ X⁽⁻⁾ (III)
where R1 to R4, independently of one another, are aliphatic groups, aromatic groups, alkoxy groups, polyoxyalkylene groups, alkylamido groups, hydroxyalkyl groups, aryl groups or alkaryl groups having 1 to 22 carbon atoms, and X⁽⁻⁾ is an anion.

10. Composition according to Claim 8, **characterized in that** the polymer with cationic or cationizable groups is chosen from methylvinylimidazolium chloride/vinylpyrrolidone copolymers, quaternized vinylpyrrolidone/dimethylaminoethyl methacrylate copolymers, cationically derivatized polysaccharides, chitosan, chitosan salts and chitosan derivatives.

11. Composition according to one of the preceding claims, **characterized in that** it additionally comprises at least one silicone compound (D) which has at least one hydrophilic group.

12. Composition according to Claim 11, **characterized in that** the hydrophilic group of the silicone compound is chosen from hydroxyl groups, primary, secondary or tertiary amino groups, quaternary ammonium groups, alkylene oxide groups, betainic groups and thiosulphate groups.

13. Composition according to Claim 11 or 12, **characterized in that** the silicone compound with at least one hydrophilic group is chosen from silicone compounds with cationic groups, hydroxy-substituted siloxanes, siloxane/polyoxyalkylene copolymers and amino-substituted siloxanes.

14. Hair-treatment agent with a content of a composition according to one of the preceding claims, **characterized in that** it is in an optically clear form.

15. Hair-treatment product consisting of a transparent or translucent packaging comprising a composition according to one of the preceding claims.

## Revendications

1. Composition pour un produit de traitement capillaire comprenant
(A) au moins un épaississant associatif non ionique, amphiphile dans une base cosmétique appropriée, l'épaississant associatif étant choisi parmi les produits de réaction de la polycondensation à catalyse acide de monomères aminoplastes au moins bifonctionnels et d'alkylènepolyéthers au moins bifonctionnels ainsi que de composés monofonctionnels ayant des groupes hydrophobes, et
(B) au moins un agent propulseur.

2. Composition selon la revendication 1, **caractérisée en ce que** les monomères aminoplastes sont choisis parmi 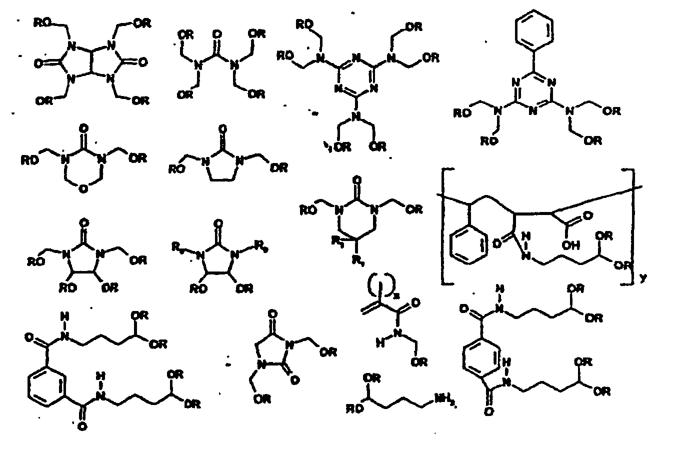 où R représente un hydrogène, un alkyle en C₁ à C₄ ou un acyle en C₁ à C₄ ; R₁ représente un alkyle en C₁ à C₄, x vaut 0 ou 1 et y vaut au moins 2 ; R₀ représente un alkyle en C₁ à C₄, un aryle ou un cycloalkyle.

3. Composition selon l'une quelconque des revendications précédentes, **caractérisée en ce que** l'épaississant est choisi parmi le groupe constitué de produits de la réaction à catalyse acide de dérivés du glycoluril et de polyalkylèneglycols et d'hydrocarbures alcoxylés.

4. Composition selon l'une quelconque des revendications précédentes, **caractérisée en ce que** l'épaississant est choisi parmi le groupe constitué de produits de la réaction à catalyse acide de (a) glycolurils de formule générale (II), 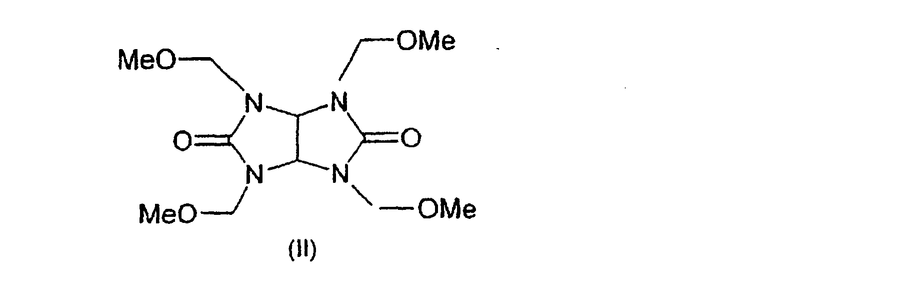 avec (b) des polyéthylène-oxyde diols d'un degré d'éthoxylation de 20 à 500 ainsi que (c) un alcool hydrophobe éventuellement éthoxylé, un alkylphénol, un thiol, un carboxamide, un carbamate ou un acide carboxylique hydrophobe.

5. Composition selon l'une quelconque des revendications précédentes, **caractérisée en ce que** l'épaississant est choisi parmi le groupe constitué du polyéther-1, du copolymère PEG-180/Octoxynol-40/TMMG et du copolymère PEG-180/Laureth-50/TMMG.

6. Composition selon l'une quelconque des revendications précédentes, **caractérisée en ce que** l'agent propulseur est choisi parmi le groupe constitué du n-butane, de l'isobutane, du propane, de l'éther diméthylique, d'hydrocarbures fluorés et de mélanges des agents propulseurs mentionnés.

7. Composition selon l'une quelconque des revendications précédentes, **caractérisée en ce qu'**elle comprend en outre au moins une substance de soins capillaires (C) renfermant au moins un groupe cationique.

8. Composition selon la revendication 7, **caractérisée en ce que** la substance cationique de soins capillaires est choisie parmi le groupe constitué de tensioactifs cationiques, de polymères renfermant des groupes cationiques ou cationisables, de protéines à dérivatisation cationique, d'hydrolysats de protéines à dérivatisation cationique et de la bétaïne.

9. Composition selon la revendication 8, **caractérisée en ce que** le tensioactif cationique est choisi parmi le groupe constitué de composés de formule (III)
N⁽⁺⁾R¹R²R³R⁴ X⁽⁻⁾ (III)
dans laquelle R1 à R4 représentent, indépendamment les uns des autres, des groupes aliphatiques, des groupes aromatiques, des groupes alcoxy, des groupes polyoxyalkylène, des groupes alkylamido, des groupes hydroxyalkyle, des groupes aryle ou des groupes alkaryle ayant de 1 à 22 atomes de carbone et X⁽⁻⁾ représente un anion.

10. Composition selon la revendication 8, **caractérisée en ce que** le polymère ayant des groupes cationiques ou cationisables est choisi parmi le groupe constitué de copolymères chlorure de méthylvinylimidazolium/vinylpyrrolidone, de copolymères vinylpyrrolidone/méthacrylate de diméthylaminoéthyle quaternisés, de polysaccharides à dérivatisation cationique, du chitosane, de sels du chitosane et de dérivés du chitosane.

11. Composition selon l'une quelconque des revendications précédentes, **caractérisée en ce qu'**elle comprend en outre au moins un composé siliconé (D) renfermant au moins un groupe hydrophile.

12. Composition selon la revendication 11, **caractérisée en ce que** le groupe hydrophile du composé siliconé est choisi parmi le groupe constitué de groupes hydroxy, de groupes amino primaires, secondaires ou tertiaires, de groupes ammonium quaternaire, de groupes oxyde d'alkylène, de groupes bétaïniques et de groupes thiosulfate.

13. Composition selon la revendication 11 ou 12, **caractérisée en ce que** le composé siliconé ayant au moins un groupe hydrophile est choisi parmi le groupe constitué de composés siliconés ayant des groupes cationiques, de siloxanes hydroxy-substitués, de copolymères siloxane/polyoxyalkylène et de siloxanes amino-substitués.

14. Produit de traitement capillaire comprenant une composition selon l'une quelconque des revendications précédentes, **caractérisé en ce qu'**il se présente sous une forme optiquement transparente.

15. Produit de traitement capillaire constitué d'un emballage transparent ou translucide comprenant une composition selon l'une quelconque des revendications précédentes.
